# EUROPEAN PATENT SPECIFICATION

(11) **EP 4 256 462 B1**
(45) Date of publication and mention of the grant of the patent: **02.09.2026**
(21) Application number: 21819610.3
(22) Date of filing: 02.12.2021
(51) Int. Cl.: G06V 10/46, G06V 20/64, G06V 10/44

(54) **PROCESS FOR IDENTIFYING A SUB-SAMPLE AND A METHOD FOR DETERMINING THE PETROPHYSICAL PROPERTIES OF A ROCK SAMPLE**
VERFAHREN ZUR IDENTIFIZIERUNG EINER UNTERPROBE UND VERFAHREN ZUR BESTIMMUNG DER PETROPHYSIKALISCHEN EIGENSCHAFTEN EINER GESTEINSPROBE
PROCÉDÉ D'IDENTIFICATION D'UN SOUS-ÉCHANTILLON ET PROCÉDÉ DE DÉTERMINATION DES PROPRIÉTÉS PÉTROPHYSIQUES D'UN ÉCHANTILLON DE ROCHE

(30) Priority: 03.12.2020 IT 202000029744
(43) Date of publication of application: 11.10.2023
(73) Proprietor: ENI S.p.A., 00144 Roma (IT)
(72) Inventor: MIARELLI, Marco, 20097 San Donato Milanese (MI) (IT)
(74) Representative: Biallo, Dario
(86) International application number: PCT/IB2021/061223
(87) International publication number: WO 2022/118234

(56) References cited:
- US-A1- 2016 307 312
- US-A1- 2017 018 096
- AL-MARZOUQI HASAN: "Digital Rock Physics: Using CT Scans to Compute Rock Properties", IEEE SIGNAL PROCES SING MAGAZINE, IEEE SERVICE CENTER, PISCATAWAY, NJ, US, vol. 35, no. 2, 1 March 2018 (2018-03-01), pages 121 - 131, XP011678895, ISSN: 1053-5888, [retrieved on 20180308], DOI: 10.1109/MSP.2017.2784459

## Description

### Field of application

The present invention relates to a process for identifying at least one sub-sample representative of a rock sample.

The present invention further relates to a method for determining the physical or petrophysical properties of a rock sample from said at least one representative sub-sample.

### Prior art

As is well known, rocks have complex heterogeneous structures with wide-ranging scales. The pores, clay and organic matter that make up rocks range in size from nanometres to millimetres.

Therefore, in order to determine the petrophysical and geological properties of soils and/or deposits, rock samples are analysed using various instruments according to different methodologies. Characteristics such as porosity, relative and absolute permeability, elastic properties, pore geometry and others are examined. These characteristics make it possible to establish the fundamental structure of rock samples and the corresponding flow capacity or transport properties, thus enabling the corresponding soils and/or deposits to be explored economically. Exploration of this kind makes it possible to identify the characteristics of the soils and thus trace possible hydrocarbon deposits or extensions of existing deposits. Of course, a different evaluation of the characteristics analysed for such an exploration makes it possible to recognize deposits of water or other substances and/or to establish other properties of the soil that may be useful in related fields such as for the evaluation of the flow rate of a soil or for volcanic evaluations.

One of the tools and a process that has become standard for analysing rock samples, typically those of small (millimetric) sizes, is DRP (Digital Rock Physics), which uses two- or three-dimensional digital images. DRP is an advantageous digital technique in many respects, but requires the use of specific instrumentation. In particular, powerful image acquisition tools (microCT, X-rays) at micrometric/nanometric scales, as well as powerful computational means for storing, analysing and solving calculations with complex numerical schemes.

Additional laboratory techniques are known which, using laboratory measurements, analyse the porosity and permeability in rock samples. It should be noted that laboratory techniques are time-consuming and costly and therefore they are generally limited to small rock samples.

In particular, rock samples used for laboratory analyses are centimetric in size and are called "plugs". Generally, plugs are rock cylinders with a diameter of 2.5 cm to 3.8 cm and a length of about 2.5 cm to 7.5 cm. Plugs are taken from the cores of larger sized or large-scale rock samples, also called core scale.

Although satisfactory in many respects, analysis using so-called laboratory techniques of plugs also has its drawbacks. Indeed, if the plugs analysed, or the sub-samples from which they are derived, are not sufficiently representative of the large-scale sample, the results obtained may identify soil characteristics that do not correspond to the actual conditions.

Other analysis techniques are also known that combine DRP with laboratory techniques and also use ML machine learning techniques which, in a multidisciplinary way, make it possible to create algorithms capable of learning from previous data appropriately stored in databases.

It is known the patent application US2016/307312A1 (Sungkorn Radompon et al) relates to a method for determining fabric of a geological sample via a multi-scale imaging for reservoir rocks.

The technical problem underlying the present invention is to select a sub-sample of rock which enables the technical characteristics of the original rock sample to be approximated in a simple and optimal manner, both in terms of time and instrumentation required, and which also enables the method for determining the petrophysical properties of the rock sample to be improved in terms of processing time and efficiency of the results obtained by reference to the known technique.

### Summary of the invention

The solution idea underlying the present invention is to recognize matching portions in the rock sample and to identify the sub-sample by including some parts of the matching portions. The invention is defined by the appended claims.

Based on this solution idea, the technical problem is solved by a process for identifying at least one representative sub-sample of a rock sample which comprises:
- digitally acquiring at a first resolution ("low resolution") a plurality of first 2D images of said rock sample;
- a rock analysis is carried out through a digital analysis of the properties of said first 2D images to determine a plurality of key points on each first 2D image and to define corresponding first processed images, wherein the key points are texture descriptors;
- an overlay, according to the direction, of said first processed images obtaining a processed digital rock sample;
- subdividing with a volumetric subdivision the processed digital rock sample into a plurality of three-dimensional digital blocks comprising a substantially similar volume;
- statistically processing and subdividing on the basis of the density of the key points in each three-dimensional digital block, two or more homogeneous classes of three-dimensional digital blocks in the digital sample, each homogeneous class defining a portion of the digital sample having three-dimensional digital blocks having similar key point densities and thus, similar rock properties;
- selecting at least one representative block for each homogeneous class;
- localizing and extracting a sub-sample from said rock sample, said sub-sample comprising at least one of said representative blocks for each homogeneous class.

The process also comprises:
- subdividing each first processed image into a plurality of regions of interest that allow the identification of the rock structure and/or rock properties or characteristics in the area surrounding each key point;
- digitally processing said first processed images to subdivide the key points into two or more groups of similar points, wherein the grouping defines a first clustered image for each first processed image, and wherein the grouping is made on the basis of a similarity of the digital properties of the first processed images in the regions of interest in proximity to the key points;
- representing said rock sample with a second digital sample superimposing, according to the direction T, said plurality of first clustered 2D images;
- performing the volumetric three-dimensional subdivision of the second digital sample defining the plurality of said 3D digital blocks.

The process also comprises:
- extracting by a selection said at least one representative block from each of said two or more homogeneous classes using statistical analysis and/or filtering methods for screening said 3D digital blocks.

The process provides a preliminary processing which digitally processes said plurality of first 2D and which determines for each first 2D a corresponding first processed 2D image; said rock analysis being performed on said plurality of first processed 2D images so as to define first processed 2D images comprising the key points or texture descriptors and corresponding regions of interest or volumes of interest surrounding said key points or texture descriptors, a subsequent grouping of said key points or texture descriptors, on the basis of the characteristics in said regions of interest or volumes of interest, identifies first clustered 2D images.

The localisation provides of mapping the at least one representative block on a digital sample subdivided into said homogeneous classes and identifying by means of an affine transformation said at least one representative block in said rock sample in order to identify said sub-sample.

Advantageously, the substantially similar value is determined with a statistical analysis through suitable processing and/or statistical algorithms of the characteristics identified by said similar key points or texture descriptors included in said 3D digital blocks and/or in that said value has a minimum volume value referred to a predefined computational accuracy.

The technical problem is also solved by a method for determining the physical or petrophysical properties of a rock sample which provides for identifying and extracting from said rock sample at least one sub-sample using the process according to the present invention, said at least one sub-sample comprising at least one representative block for each of the two or more homogeneous classes of three-dimensional digital blocks, and digitally acquiring, at a second resolution higher than said first resolution ("high resolution"), a plurality of third digital images of said at least one sub-sample at said at least one representative block, and performing a physical or petrophysical analysis of said plurality of third digital images acquired to determine the physical or petrophysical properties of the rock sample starting from the properties of the at least one representative block for each of said two or more homogeneous classes.

The technical problem is also solved by a sub-sample obtained from the process according to the present invention comprising at least two representative blocks having a substantially similar volume.

The technical problem is also solved by a data processing system according to claim 9 and a computer program according to claim 10.

The characteristics and advantages of the process and method according to the invention will become clear from the description, made below, of an embodiment given by way of non-limiting example with reference to the attached drawings.

### Brief description of the drawings

With reference to these figures,
- Figure 1 illustrates schematically, in a block diagram, a process and a method according to the present invention;
- Figures 2 to 11 schematically illustrate a sequence of steps in the process of Figure 1, in one embodiment;
- Figure 12 schematically illustrates the identification of the sub-sample according to the present invention from a digital sample;
- Figure 13 schematically illustrates an original sample and a sub-sample identified by the process according to the present invention;
- Figures 14 to 17 schematically illustrate certain steps and results of a test performed on a rock sample by the process and method according to the present invention.

### Detailed description

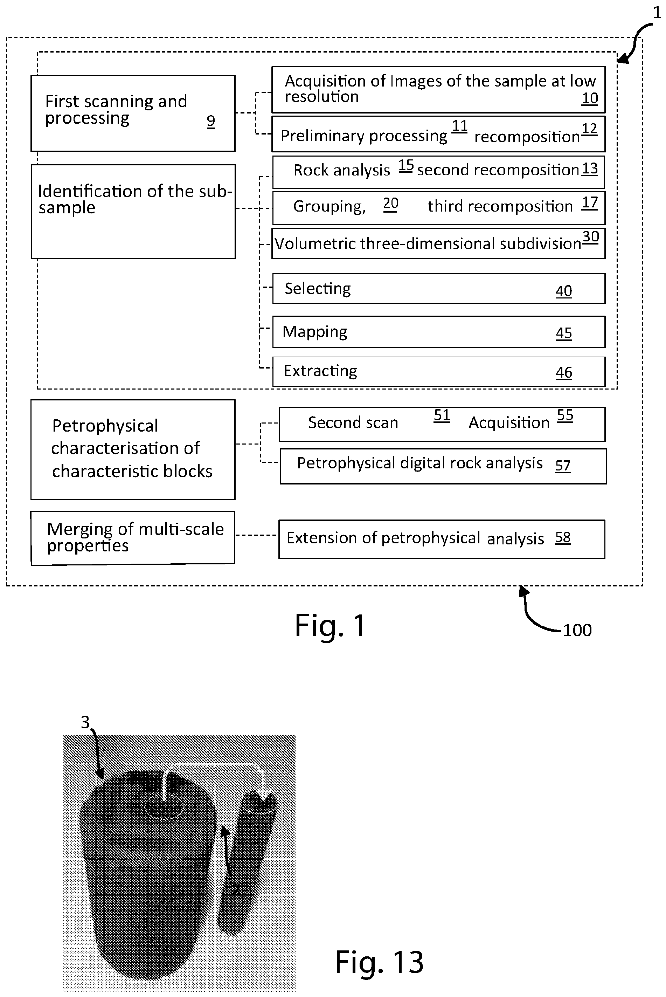

With reference to Figure 1, a process to identify a sub-sample 2, representative of a rock sample 3, is indicated in its entirety by the number 1. The sub-sample 2 is adapted to determine the petrophysical properties of the rock sample 3.

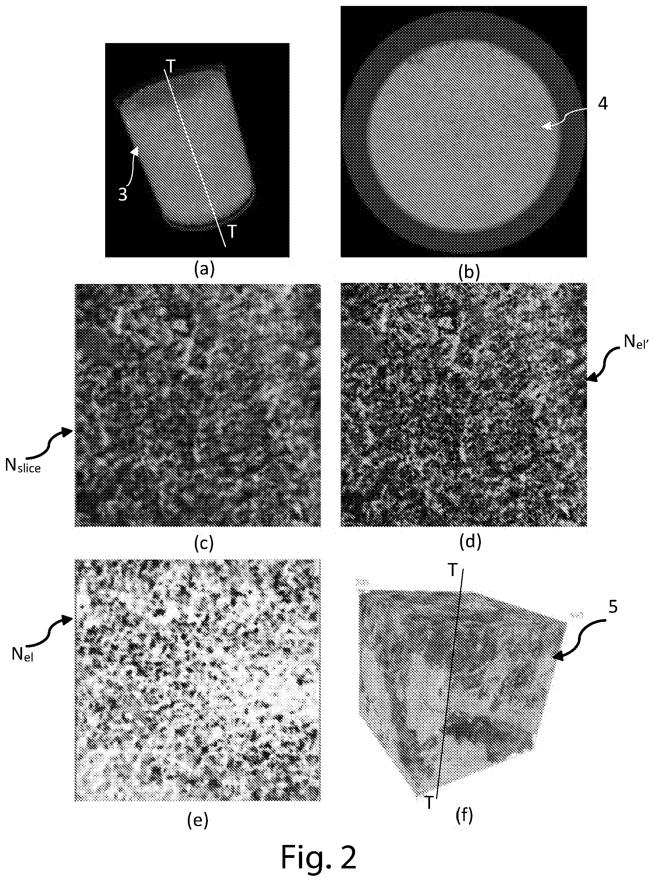

In one embodiment, the process 1 comprises a first scan 9 of the rock sample 3 to digitally acquire 10 a plurality of first images N_{slice}, as illustrated in Figure 2c. The plurality of first images N_{slice} are two-dimensional digital images and the first scan 9 is of the low-resolution, centimetre-scale type. In the present case, the digital acquisition 10 has a resolution in the range [15-40] µ-pixel. Preferably, the first scan 9 is performed on the entire rock sample 3 using a tomograph, not shown in the figures.

Each of the first images N_{slice}, in two dimensions, represents a corresponding slice 4 of the rock sample 3 whose height is substantially one pixel, at least in an indicative and non-limiting embodiment.

The plurality of first images N_{slice} is adapted to represent a digital sample 5 of rock sample 3, as for example shown in Figure 2f.

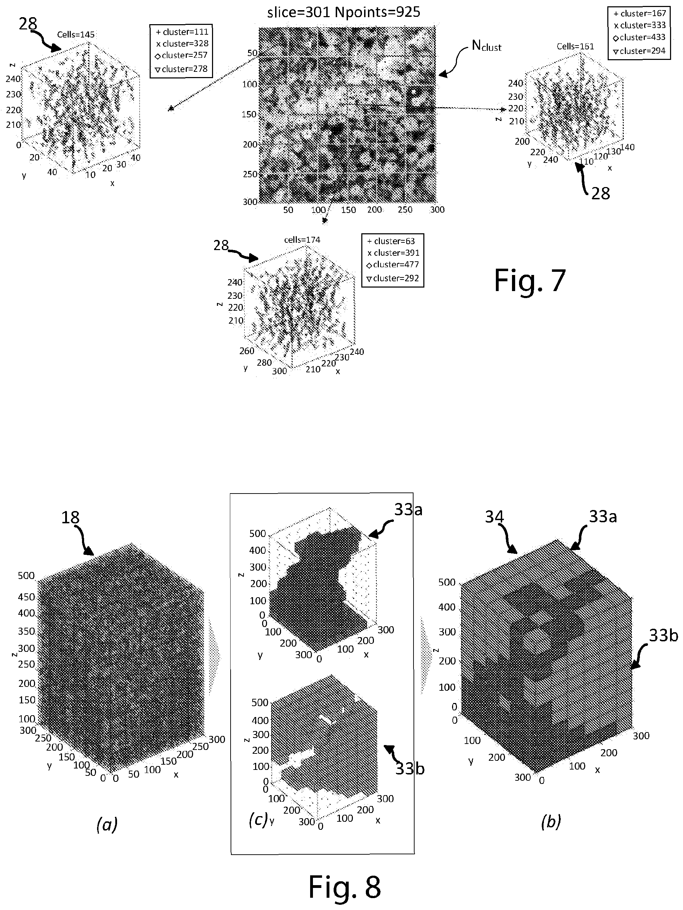

In the most general embodiment, the process 1 involves subdividing the digital sample 5 into two or more classes or homogeneous portions 33 of three-dimensional 3D digital blocks 28, as illustrated schematically in Figures 8a-8c. The 3D digital blocks 28 are equivalent to each other in terms of technical rock characteristics and comprise a substantially similar volume V of value V1.

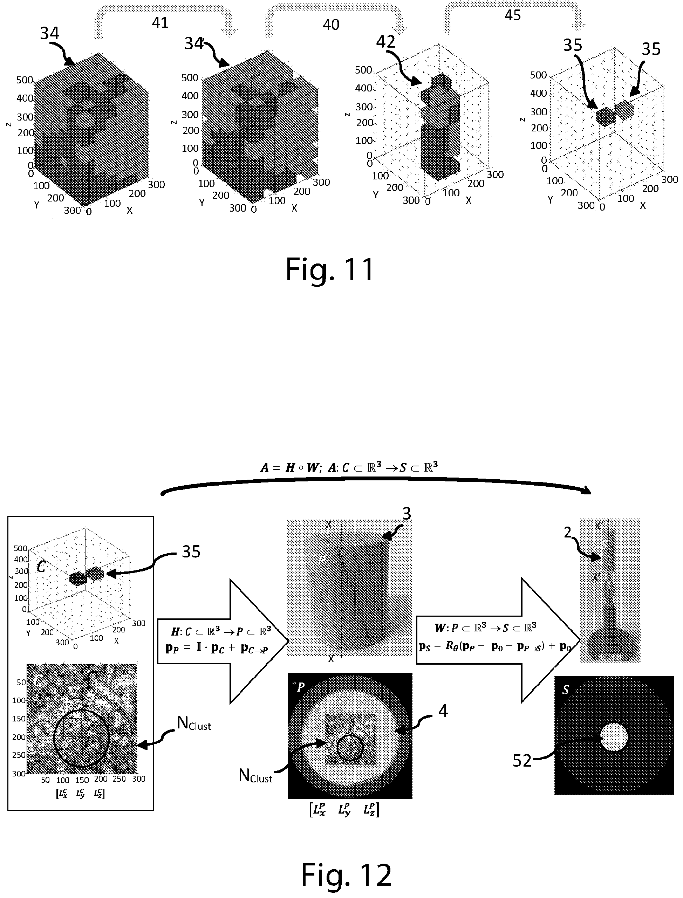

The process 1 therefore involves extracting 46 the sub-sample 2 localizing at least one representative block 35 of rock for each homogeneous class 33 identified, as shown in Figure 11 in which two homogeneous classes have been identified.

In one embodiment, illustrated in the Figures, a preliminary processing 11 of the plurality of first images N_{slice}, on the basis of the digital properties, allows the removal of noise, improvement in contrast and adjust of the intensity by determining for each first image N_{slice} a corresponding processed first image Nₑₗ. Preliminary processing 11 is carried out with analysis and processing software. Such software can basically be of the conventional type and known to a person skilled in the art. A result of the processing is shown in figure 2e, while figure 2d represents the processed image Nₑₗ at an intermediate stage of processing, processed image N_{el'}.

Preliminary processing 11 may also involve cutting out the perimeter edges of said corresponding slice 4 obtaining for each first image N_{slice} a substantially square area, as illustrated in Figure 4c.

A recomposition 12 of said first processed images Nₑₗ, superimposed according to a directrix T, allows a digital sample 5 also called Cropped Digital Core Plug (CDCP) of the rock sample 3 to be obtained, as illustrated in Figure 2f in a perspective view.

The digital sample 5 comprises a number of pixels that is defined by ${L}_{x}^{C} \times {L}_{y}^{C} \times {L}_{z}^{C}$

Furthermore, considering the plurality of first images processed Nₑₗ, the digital sample 5 can be represented by the expression: $\left\{{M}_{{L}_{x}^{C} \times {L}_{y}^{C}}\right\} i ∈ l$ wherein $I = \left\{1,….{N}_{slices}={L}_{z}^{C}\right\}$

Each first image N_{slice} and each corresponding first processed image Nₑₗ highlight rock features: pores (empty) or rock (solid) that make up each corresponding slice 4. Thus, the rock characteristics of rock sample 3 are also represented in said digital sample 5.

Subsequently, a Rock-Typing or rock analysis 15 is carried out through a digital analysis of the properties of said first processed images Nₑₗ.

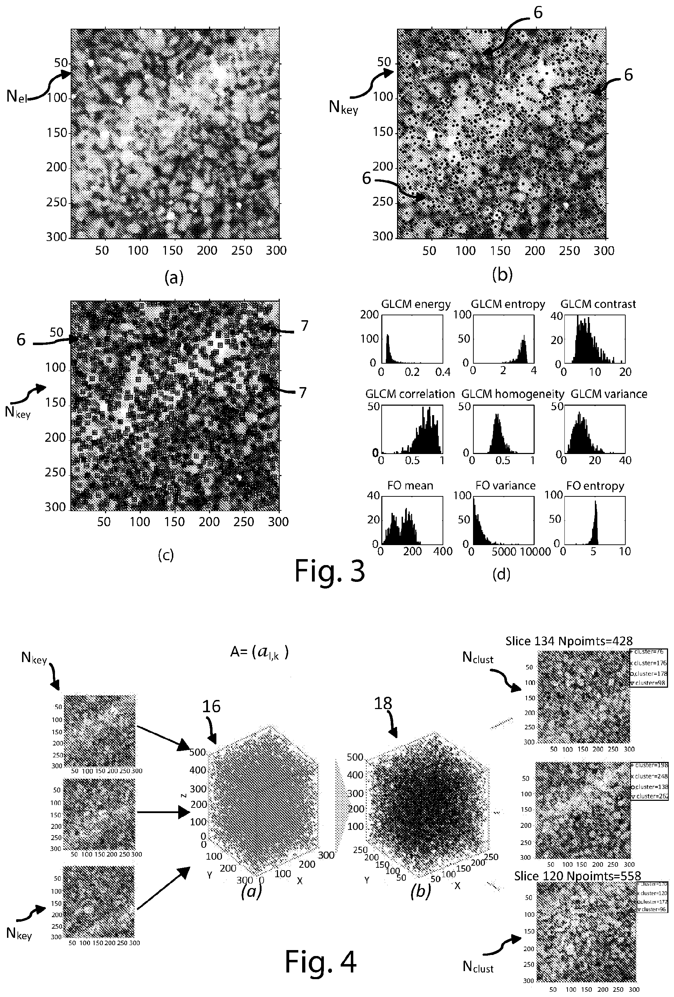

The rock analysis 15 determines a plurality of key points 6 on each first processed image Nₑₗ and defines corresponding first processed images N_{key}, as illustrated schematically in Figures 3a and 3b. The key points 6 are texture descriptors and are defined in a variable number for each first processed image N_{key}.

With the use of additional processing and editing software, the key points 6 allow each first processed image N_{key} to be subdivided into a plurality of regions of interest 7, as illustrated in Figure 3c. Each region of interest 7 allows the identification of the rock structure and/or rock properties or characteristics in the area surrounding each key point 6.

Through processing and elaboration of each first processed image N_{key}, it is possible to numerically describe the properties of the rock structure at the regions of interest 7. Such properties are extracted and calculated from the characteristics of the analysed digital image. For example, analysis of appropriately defined histograms (FO) and calculation and analysis of the grey level co-occurrence matrix (GLCM) . Indicatively, as schematically illustrated in Figure 3d, the following values can be obtained: GLCM energy, GLCM entropy, GLCM contrast, GLCM correlation, GLCM homogeneity, GLCM variance, FO mean, FO variance, FO entropy. Then, from these values it is possible to derive the digital properties associated with the different degrees of porosity of the rock.

Further digital processing of said first processed images N_{key}, by means of a grouping or cluster 20 of said key points 6, allows the key points 6 to be subdivided into two or more groups 8 of similar points, as illustrated in Figures 4a and 4b. The grouping 20 is made on the basis of a similarity of the digital properties of said first processed images N_{key} in the regions of interest 7 in proximity to said key points 6. With the grouping 20 each first processed image N_{key} defines a first clustered image N_{Clust}.

In fact, each group of similar points 8 creates a link between the characteristics of the rock structure and the regions of interest 7 identifying image classes with similar or related properties.

The number of such groups 8 of similar points depends in general on the heterogeneity of the rock in said first processed images N_{key}.

The grouping 20, both for its execution and for the determination of the number of groups 8, uses supervised or non-supervised algorithms which, according to one embodiment, may also include machine learning methodologies. The rock analysis 15 and grouping 20, by way of non-limiting example, may use software such as Scale Invariant Feature Transform (SIFT) .

A second recomposition 13 with an overlay, according to the directrix T, of the first processed images N_{key} allows the rock sample 3 to be represented as a processed digital rock sample 16, illustrated in Figure 4a. In one embodiment, the processed digital rock sample 16 is described by a Matrix A of the type: $Matrix A = \left({a}_{lk}\right) ∈ {ℝ}^{2}$ where k identifies the number of characteristics chosen, while the value l is obtained from the formula: $l = i + {\sum }_{j = 1}^{p - 1} {N}_{key} \left(j\right) where i = 1 , … , {N}_{key} \left(p\right) and p = 1 , … , {N}_{slices}$

A third recomposition 17 of said first clustered images N_{Clust}, according to the directrix T, allows a second digital rock sample 18 to be defined, schematically illustrated in Figure 4b.

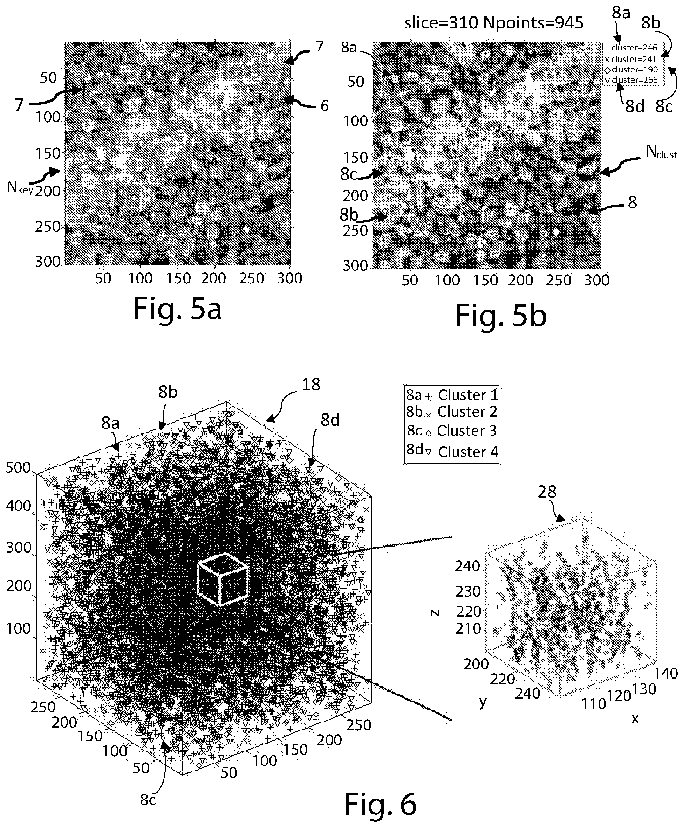

In the illustrated embodiment, the key points 6 are grouped into four groups, 8a-8d, also called clusters, as illustrated in Figures 4b, 5b and 6.

In the second digital rock sample 18 each group 8 identifies related or corresponding rock classes.

Advantageously, the process 1 provides for a volumetric three-dimensional subdivision 30 of said second digital sample 18 by defining a plurality of said 3D digital blocks 28 equivalent to each other by volume V. Each 3D digital block 28 comprises a value V1 of volume V.

Preferably, the volume value V1 is determined by a statistical analysis 25 through appropriate processing and/or statistical algorithms of the characteristics identified by said groups 8a-8d of similar points included in said 3D digital blocks 28. According to an alternative embodiment, the volume value V1 is a predefined value.

Naturally, each 3D digital block 28 of volume V1 comprises classes of two or more first clustered images N_{clust} with a different key point density 6 for each 8a-8d group, as shown in Figure 7.

Statistical processing 32 of said plurality of 3D digital blocks 28 results in homogeneous classes 33 of blocks. The 3D digital blocks 28 of each homogeneous class 33 have substantially similar key point densities 6 and thus, advantageously, similar rock properties i.e. similar petrophysical properties at least in the described embodiment.

The rock property data as shown above of said 3D digital blocks 28 of volume V1 can be arranged in a matrix $matrix B = \left({b}_{n , p}\right) ∈ {ℝ}^{2} ′$

Wherein:
n= 1,....N_{REV} (p) represents the number of blocks 28 that make up the second rock sample 18;
p the number of features that can be assigned to or searched for in each 3D digital block 28.

In the illustrated embodiment, by performing one or more supervised machine learning methods on the matrix B, the 3D digital blocks 28 are subdivided by defining two homogeneous classes 33a and 33b, as illustrated in Figure 8c. According to a preferred but not limiting embodiment, the statistical processing 32 involves the use of the Kmean method (Arthur D, Vissilvitskii S (2007) K-means++: the advantages of careful seeding. Proc of the Annual ACM-SIAM Symposium on Discrete Algorithms 8:1027-1035, DOI 10.1145/1283383.1283494).

In an implementation of the process 1, the density of the key points 6 in each 3D digital block 28 of volume V1 is used as one of the statistical parameters to determine the number of homogeneous classes 33.

In addition, each 3D digital block 28 must have at least a predefined minimum Min volume value V1 necessary for computational accuracy referring to an average quantity. According to one embodiment, the 3D digital block 28 has so-called REV dimensions for a minimum Mₘᵢₙ value of volume V1.

In a non-limiting example, the size of a REV block can be in the range of 0.5-1.7 mm³ preferably 1 mm³, or obtained according to the article by Mostaghimi et al. (2012-Computations of absolute permeability on micro-ct images; Mathematical Geosciences 45, DOI 10.1007/s1 1004-012-9431-4). Alternatively, these dimensions can be obtained according to the article by Al-Raoush R. and Papadopoulos A. (2010-Representative elementary volume analysis of porous media using X-ray computed tomography. Powder Technology 200:69-77, DOI 10.1016/j.powtec.2010.02.011) or Nordahl K. and Ringrose P (2008-Identifying the representative elementary volume for permeability in heterolithic deposits using numerical rock models. Mathematical Geosciences 40:753-771).

The rock sample 3 is then represented as a third rock sample 34 subdivided into homogeneous classes 33 of equivalent blocks appropriately interfaced with each other, as illustrated in Figures 8a-8c.

In the example shown in the intermediate frame of Figure 8, the third rock sample 34 has two homogeneous classes, 33a and 33b, of equivalent blocks.

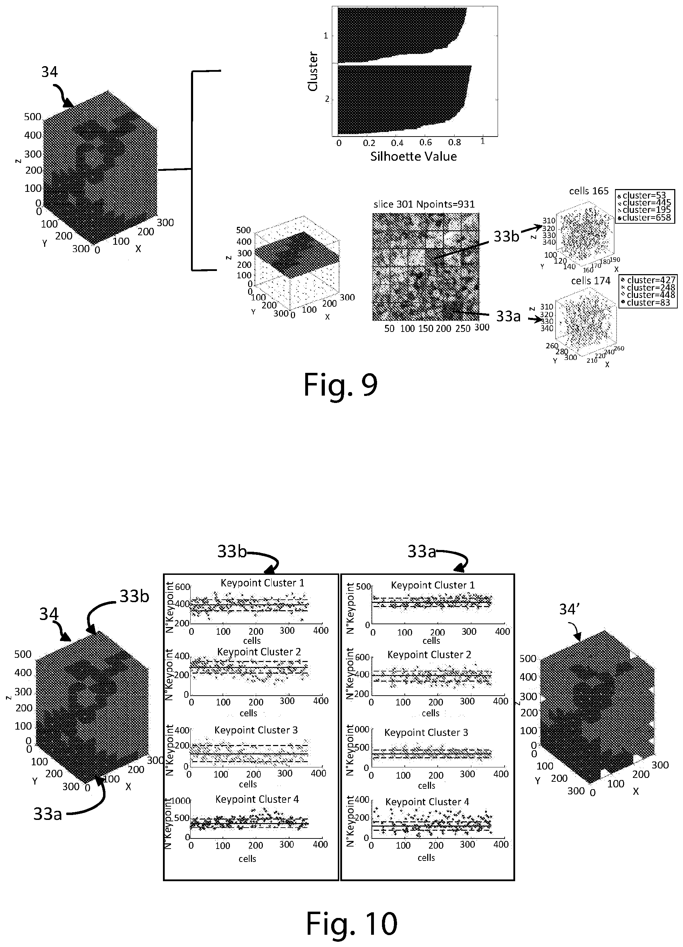

In one embodiment, it is possible to evaluate the goodness of the three-dimensional 30 volumetric subdivision and statistical processing 32 by using silhouette graphs (Rousseeuw PJ, 1987- Silhouettes: A graphical aid to the interpretation and validation of cluster analysis. Journal of Computational and Applied Mathematics 20:53-65, DOI https://doi.org/10.1016/03777-0427(87)90125-7) and by visual comparison as shown schematically in Figure 9.

Next, the process 1 involves selecting 40 one or more representative blocks 35 from each homogeneous class, 33a and 33b, identified.

In one embodiment, the selection 40 provides for extracting said representative blocks 35 also using statistical methodologies and/or appropriate filtering methods for further screening of said 3D digital blocks 28 allowing for improved identification.

In an indicative and non-limiting embodiment, the selection 40 uses an IOR method of iterative outlier removal, illustrated schematically in Figure 10. In Figure 10, the solid/broken black line represents the mean/variance of convergence of the algorithm and the graphs shown are for the two homogeneous classes 33a and 33b identified.

In one embodiment, it is possible to use the IOR method as developed in "Python script" and described for example by Clewlow L. and Strickland C. (2000-Energy derivatives: Princing and risk management) and/or by Parrinello CM et al (2016-Interactive Outlier Removal: A method for Identifying Outliers in Laboratory Recalibration Studies. Clinical Chemistry 62(7):966-972 DOI 10.1373/clinchem.2016.255216).

In essence, the IOR method or analogues allow a screening 41 of said digital blocks-3D 28 equivalent in said homogeneous classes, 33a and 33b-and to define a third screened rock sample 34'.

According to one embodiment, the selection 40 using the IOR method involves successively:
1) measuring the mean and standard deviation of the key points 6 clustered in each equivalent block 28 belonging to a homogeneous class 33;
2) removing all the equivalent blocks 28 having a key point density 6 greater than the mean value plus two times the value of the standard deviation;
3) repeating the measurement and removal until the convergence that defines a group of candidates 42.

Subsequently, one or more groups of candidates 35 are chosen from one or more representative blocks 42 by applying further algorithms and/or based on physical constraints related to the size of the original rock sample 3 and/or related to the methodology of breaking the original rock sample 3, as schematically illustrated in Figure 11.

Thus, mapping 45 is performed, localizing the representative blocks 35 from the group of candidates 42 in said third rock sample 34. A suitable extraction 46 allows the extraction of a sub-sample 2, of the rock sample 3, comprising one or more representative blocks 35 for each group of candidates 42.

By means of an affine transformation involving a linear translation and a rototranslation, as illustrated in Figure 12, it is possible to map 45 the sub-sample 2 with the location of one or more representative blocks 35 for each homogeneous class, 33a and 33b.

In one embodiment, the affine transformation of the rock sample 3 which can be described by: $A = H ∘ W ; A : C ⊂ {\overline{ℝ}}^{3} \to S ⊂ {ℝ}^{3}$
which basically results from a combination of a linear transformation $H : C ⊂ {ℝ}^{3} \overline{\to } P ⊂ {ℝ}^{3} ′$
for the transformation of the coordinates from the third rock sample 34 to the rock sample 3 according to the equation: $H \left({p}_{C}\right) = {p}_{p} = {T}_{v} \left({p}_{C}\right)$
and then a rototranslation to transform the coordinates from the rock sample 3 to the sub-sample 2, according to the equation: $W \left({p}_{p}\right) = {p}_{s} = {R}_{θ} \left({p}_{p}-{p}_{0}-{p}_{i , P \to S}\right) - {p}_{0}$
wherein
p_{c =} (x_{c}, Y_{c}, z_{c}) represents the coordinates of the points on a reference system relative to the third rock sample 34;
p_{s =} (xₚ, Yₚ, zₚ) represents the coordinates of the points on a reference system relative to the rock sample 3;
**T*ᵥ*** is the translation matrix of the component $ν = \left(0.5\times \left({L}_{x}^{P}-{L}_{x}^{C}\right)-1,0.5\times \left({L}_{y}^{P}-{L}_{y}^{C}\right)-1,0.5\times \left({L}_{z}^{P}-{L}_{z}^{C}\right)-1\right) .$
***pₛ* = (*x_{S}*,*y_{S}*,*z_{S}*)** represents the coordinates of the points on a reference system relative to the sub-sample 2;
${p}_{0} = \left({L}_{x}^{P}/2,{L}_{y}^{P}/2,{L}_{z}^{P}/2\right)$ represents the coordinates of the point of origin of the rotation in the reference system relative to rock sample 3;
***p_{P→S} =* (*x_{P→S}*, *y_{P→S}*, *z_{P→S}*)** is the vector transformation from the vector P to the vector S,
***θ*** represents the angle of rotation;
**R*_{θ}*** is the inverse matrix of the standard rotation.

In one embodiment, the coordinate system can be obtained through the method described by Sok R. et al. (2010-Pore scale characterization of carbonates at multiple scales: Integration of micro-ct, bsem, and fibsem. Petrophysics 51) by relating the pixels of the images defining the third rock sample 34 to the coordinates of the points in the rock sample 3. In particular, the method uses predefined base points that localize known points in the rock sample 3, as illustrated schematically in Figure 12.

In one embodiment, the representative blocks 35 are preferably selected substantially close together due to requirements related to the operation of cutting the rock sample 3. A cylinder comprising or corresponding to said sub-sample 2 may be identified with an axis X' substantially parallel to an axis X of the rock sample 3, as illustrated in Figure 12.

The extraction 46 of the sub-sample 2 is an invasive operation for the rock sample 3, which is cut and then destroyed. As is clear to a person skilled in the art, the process 1 could identify two or more sub-samples 2 of the rock sample 3, depending on the size of the rock sample 3 and the type of analysis required.

The process 1 as described allows the rock sample 3 to be divided into homogeneous classes or homogeneous classes 33 of blocks that are equivalent to each other in terms of technical rock characteristics and to extract the sub-sample 2 by localizing one or more representative blocks 35 of rock for each identified homogeneous class 33. It was found that the process 1 according to the present invention substantially and significantly reduces the processing time for identifying and extracting the sub-sample adapted to determine the petrophysical properties of the rock sample 3.

According to a further aspect of the present application, a method for determining the petrophysical properties 100 of a rock sample 3 is now described.

The method involves extracting a sub-sample 2 according to the process 1 described above, and parts and classes having the same structure and function will be given the same numbering and reference code.

A second digital scan 51 of the sub-sample 2 allows for the high-resolution digital acquisition 55 of a plurality of second digital images 52 at said representative blocks 35. In the present case, the digital acquisition 55 has a resolution in the range [1.5-2.5] µ-pixel. The digital acquisition 55 is carried out through a targeted micro-tomographic acquisition at said representative samples 35 for each homogeneous class 33.

Subsequently, a petrophysical analysis 57 of rock, of the plurality of second digital images 52 acquired allows the petrophysical properties of each representative sample 35 to be identified.

Thus, the petrophysical properties identified in said representative blocks 35 are extended 58 to the plurality of 3D digital blocks 28, according to the corresponding homogeneous class, 33a and 33b, of the third rock sample 34.

In one embodiment, the petrophysical analysis 57 of sub-sample 2 can be carried out
using a so-called CFD-type solver that characterizes the micrometer-scale fluid flow details of selected samples by constructing a computational grid from 3D micrometric images (3D micro-CT images), as for example described by Blunt M. et al (2013- Pore-scale imaging and modelling. Advances in Water Resources 51:197-216, DOI 10.1016/j.advwatres.2012.03.003) and by Mostaghimi et al. (2012).

For the determination of petrophysical properties, the presence of microporous zones plays an important role in defining the connectivity between pores and thus in determining the final permeability. Therefore, by way of non-limiting indication, in order to determine a microporosity, e.g. in the presence of porous structures with pore sizes smaller than the voxel size of the image, the petrophysical analysis 57 of the sub-sample 2 can be carried out through an algorithm described by Verri et al. (2017-Development of a digital rock physics workflow for the analysis of sandstones and tight rocks. Journal of Petroleum Science and Engineering 156:790-800, DOI https://doi.org/10.1016/j.petrol.2017.06.053).

Furthermore, the effect of microporosity in a representative block 35, in which porous regions can be included in the pore space, is modelled through equations that start from conservation of mass and momentum and describe the fluid-dynamic properties with increased fluid resistance. These equations are: $v ⋅ u = 0$ $∇ ⋅ \left(ρuu\right) = ρ g - μ {∇}^{2} u + R$ Wherein:
***u*** is the velocity of the fluid
***P*** is the density of the fluid
µ is the kinematic viscosity of the fluid
g is the acceleration of gravity
**∇** Represents the nabla differential operator.

R is the resistivity source term calculated as the mean level of the greys corresponding to the image voxels in the microporous regions identified in said plurality of third, high-resolution, digital images 52.

According to other embodiments, the petrophysical analysis 57 may require subjective evaluations of said second digital images 52.

In one embodiment, a simulated global flow analysis 59 is applied to the second rock sample 34. According to a fluid dynamic approach, for each isothermal phase, with a steady state of the incompressible Newtonian fluid, conservation of mass and momentum is considered, and thus the fluid flowing through the porous mass is described by the Darcy equations: $∇ ⋅ u = 0$ $u = - \frac{K}{μ} \left(∇ρ-ρg\right)$ Wherein:
***u*** is the velocity of the fluid
***p*** is the density of the fluid
P is the pressure
µ is the kinematic viscosity of the fluid
g is the acceleration of gravity
**∇** Represents the nabla differential operator.

With the simulated global flow analysis 59 it is then possible to define the petrophysical properties of the rock sample 2.

The present invention also relates to a data processing system comprising a tomograph configured to perform a first digital scan 9, at low resolution, on a rock sample 3 by defining a plurality of first images N_{slice}. The system further comprises a processor configured to acquire said plurality of first images N_{slice} and to perform the process 1 to identify a sub-sample 2 representative of a rock sample 3, as described above.

In addition, the system comprises a further tomograph configured to perform a second, high-resolution digital scan 51 of a sub-sample 2 of a rock sample 3. Said processor or a further processor being configured to perform the method 100 to determine the petrophysical properties of the rock sample 3 as described above.

Furthermore, the present invention comprises a computer program having instructions which, when the program is run by a computer, the computer performs the process of identifying a representative sub-sample 2 of a rock sample 3, as described above. Such program further comprising instructions such that when the program is run by a computer, the computer performs the method 100 to determine the petrophysical properties of the rock sample 3 as set forth above.

Advantageously, the process to determine the sub-sample, the method to determine the petrophysical or physical properties of the rock sample, the system and the program, as described, make it possible to compare the petrophysical or physical properties from a DRP scale to a laboratory scale in a quick and optimal way, both in terms of processing time and in terms of the instrumentation required, as is clear to a person skilled in the art.

By means of an initial low-resolution tomographic acquisition, areas of heterogeneous samples are identified guiding the choice of homogeneous classes of rock samples.

A second high-resolution tomographic acquisition allows the absolute permeability of the rock sample to be assessed from the petrophysical or physical properties measured in the individual representative samples.

### Tests performed

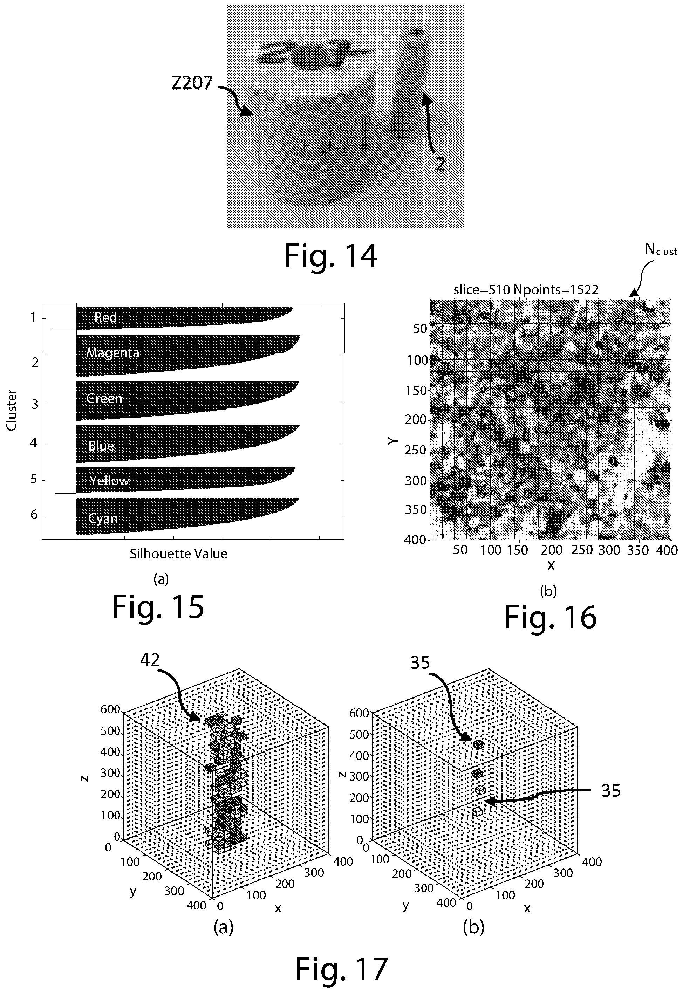

It was possible to evaluate the goodness of the process and method described through some tests using a rock sample 3, called Z207, shown in Figure 14, and the sub-sample 2 obtained through the process 1, described above.

It was possible to compare the simulated flow analysis 59 on a third rock sample 34, not shown in the figures, obtained from Z207, with a real flow analysis 58. Z207 has a diameter of 38 mm and a height of 50 mm with voxel dimensions (Lₓ^{P}xLₓ^{P}xLₓ^{P}) = (1004x1024x1014) . Z207 is composed of heterogeneous carbonate rock with some fossil fragments. Figure 15 shows some first images N_{slice} of the Z207 and preliminary processing provides first processed images Nₑₗ of dimensions (L_{X}^{P}xL_{X}^{P}xL_{X}^{P}) = (400x400x600) . With the rock analysis 15 applied to the 600 images processed Nₑₗ one at a time, the average number of key points 6 was about 1400 per image. In order to obtain a good representation of rock characteristics, the grouping 20 provides six distinct groups 8a-8f of similar points. Figure 16 shows the analysis, with Silhouette graphs, for the six distinct groups. With the combination of FO entropy and mean FO, it is possible to estimate the pixels tones in the regions of interest 7 that surround each key point 6 of each image. A clustered image N_{Clust} is shown in Figure 17. Three-dimensional volumetric subdivision 30 subdivides the second rock sample 18 defining 12000 3D digital blocks 28 with a volume V1 of (20x20x20) voxels corresponding to (1.19x1.19) mm. An analysis of said 3D digital blocks 28, using appropriate algorithms, determined two homogeneous classes, 33a and 33b, for Z207.

The selection 40 and localization of representative blocks 35 is illustrated in Figures 17a-b.

The sub-sample 2 with a diameter of 5 mm and a length of 50 mm was obtained by extraction, illustrated in Figure 14.

Thus, according to the method for determining the petrophysical properties, the high-resolution acquisition of the sub-sample 2 makes it possible to obtain a plurality of third images 52 from which the petrophysical properties of Z207 can be determined.

The simulated flow analysis 59 tested uses conventional methodologies such as flow-based methods, e.g. Durlofsky L (2005-Upscaling and gridding of fine scale geological models for flow simulation. Paper presented at the International Forum on Reservoir Simulation Iles Borromees, Stresa, Italy, June 20-24) .

Simulated flow analysis 59 was carried out with a uniform fixed velocity at the inlet and a path flowing along a longitudinal direction of the third rock sample 34 divided into the two homogeneous classes, 33a and 33b.

The simulated analysis was compared with a real flow analysis performed on the rock sample Z207 in the Z-direction substantially perpendicular to the bearing plane of Z207. The absolute permeability values defined with the simulated flow analysis 59 are comparable with the values obtained in the laboratory with the real flow analysis 58 confirming the goodness of the process and method as described.

It has been possible to observe that the process described to identify a representative sub-sample of a rock sample achieved the predefined objects. In particular, a person skilled in the art can see how the process, method and system described allow a comparison between digital and experimental data, enabling petrophysical or physical properties to be obtained at the same scale.

It has also been observed that the process, method and system described are also suitable for use in the presence of rock samples that are deficient due to non-consolidation and/or fracture.

## Claims

1. A process (1) for identifying a sub-sample (2) representative of a rock sample (3), which comprises
- digitally acquiring (10) at a first resolution a plurality of first 2D images (N_{slice}) of said rock sample (3);
- a rock analysis (15) is carried out through a digital analysis of the properties of said first 2D images to determine a plurality of key points (6) on each first 2D image and to define corresponding first processed images (N_{key}), wherein the key points (6) are texture descriptors;
- an overlay, according to the direction (T), of said first processed images (N_{key}) obtaining a processed digital rock sample (16);
- subdividing with a volumetric subdivision (30) the processed digital rock sample (16) into a plurality of three-dimensional digital blocks (28) comprising a substantially similar volume;
- statistically processing and subdividing on the basis of the density of the key points in each three-dimensional digital block, two or more homogeneous classes of three-dimensional digital blocks in the digital sample, each homogeneous class (33, 33a and 33b) defining a portion of digital sample (34) having three-dimensional digital blocks (28) with similar density of key points (6) and thus similar rock properties;
- selecting at least one representative block (35) for each homogeneous class (33, 33a and 33b);
- localizing and extracting (46) a sub-sample (2) from said rock sample (3), said sub-sample (2) comprising at least one of said representative blocks (35) for each homogeneous class (33, 33a and 33b).

2. The process (1) according to claim 1, **characterized in that** it comprises:
- performing a further rock analysis subdividing each first processed image (N_{key}) into a plurality of regions of interest (7) that allow the identification of the rock structure and/or rock properties or characteristics in the area surrounding each key point (6);
- digitally processing said first processed images (N_{key}) to subdivide the key points (6) into two or more groups (8, 8a and 8b) of similar points, wherein the grouping (20) defines a first clustered image (N_{Clust}) for each first processed image (N_{key}), and wherein the grouping (20) is made on the basis of a similarity of the digital properties of the first processed images (N_{key}) in the regions of interest (7);
- representing said rock sample (3) with a second digital sample (18) superimposing, according to the direction T, said plurality of first clustered images (N_{Clust});
- performing the volumetric subdivision (30) of said second digital sample (18) defining the plurality of said three-dimensional digital blocks (28).

3. The process according to claim 1 or 2, **characterized by**:
- extracting by a selection (40) said at least one representative block (35) from each of said two or more homogeneous classes (33, 33a and 33b) using statistical analysis and/or filtering methods for screening said 3D digital blocks (28).

4. The process according to claim 3, **characterized by** providing for a preliminary processing (11) which digitally processes said plurality of first 2D images (N_{slice}) and which determines for each first 2D image (N_{slice}) a corresponding first processed 2D image (Nₑₗ), said rock analysis (15) being performed on said plurality of first processed 2D images (Nₑₗ).

5. The process according to claim 1, **characterized in that** the localizing comprises mapping (45) said at least one representative block (35) on a digital sample (34, 34') subdivided into said homogeneous classes (33, 33a and 33b) and identifying by means of an affine transformation said at least one representative block (35) in said rock sample (3) in order to identify said sub-sample (2).

6. The process according to claim 1, **characterized in that** said substantially similar volume is determined with a statistical analysis (25) of the density of said key points (6) included in said three-dimensional digital blocks (28) or **in that** said substantially similar volume has a minimum volume value (V1ₘᵢₙ) referred to a predefined value.

7. A method (100) for determining the physical or petrophysical properties of a rock sample (3) **characterized by**:
- identifying and extracting from said rock sample (3) at least one sub-sample (2) using one or more of claims 1 to 6, said at least one sub-sample (2) comprising at least one representative block (35) for each of the two or more homogeneous classes (33, 33a and 33b) of three-dimensional digital blocks (28);
- digitally acquiring (55), at a second resolution higher than said first resolution, a plurality of third digital images (52) of said at least one sub-sample (2) at said at least one representative block (35);
- performing a physical or petrophysical analysis (57) of said plurality of third digital images (52) acquired to determine the physical or petrophysical properties of said rock sample (3) starting from the properties of said at least one representative block (35) for each of said two or more homogeneous classes (33).

8. A sub-sample (2) obtained from a process according to one or more of claims 1 to 6, **characterized in that** it comprises at least two representative blocks (35), said at least two representative blocks (35) having substantially similar volume.

9. A data processing system **characterized by** comprising:
- a tomograph configured to perform a first digital scan (9) at a first resolution on a rock sample (3) defining a plurality of first 2D images (N_{slice}), and
- a processor configured to acquire said plurality of first 2D images (N_{slice}), and to execute the process (1) for identifying a sub-sample (2) representative of said rock sample (3) according to one or more of the claims 1 to 6, and/or comprising:
- a further tomograph configured to execute a second digital scan (51) at a second resolution higher than the first resolution of the sub-sample (2) defining a plurality of third 2D images (52), and
- a further processor or said processor configured to execute the method for determining the physical or petrophysical properties of a rock sample (3) according to claim 7.

10. A computer program **characterized in that** it comprises instructions which when the program is executed by a computer, the computer executes the process according to one or more of claims 1 to 6 and/or executes the method according to claim 7.

## Patentansprüche

1. Verfahren (1) zum Identifizieren einer für eine Gesteinsprobe (3) repräsentativen Teilprobe (2), welches umfasst:
- digitales Erfassen (10) einer Vielzahl von ersten 2D-Bildern (N_{slice}) der Gesteinsprobe (3) mit einer ersten Auflösung;
- eine Gesteinsanalyse (15) wird durch eine digitale Analyse der Eigenschaften der ersten 2D-Bilder durchgeführt, um eine Vielzahl von Schlüsselpunkten (6) auf jedem ersten 2D-Bild zu bestimmen und entsprechende erste verarbeitete Bilder (N_{key}) zu definieren, wobei die Schlüsselpunkte (6) Texturdeskriptoren sind;
- eine Überlagerung der ersten verarbeiteten Bilder (N_{key}) entsprechend der Richtung (T), wodurch eine verarbeitete digitale Gesteinsprobe (16) erhalten wird;
- Unterteilung der verarbeiteten digitalen Gesteinsprobe (16) mittels einer volumetrischen Unterteilung (30) in eine Vielzahl von dreidimensionalen digitalen Blöcken (28), die ein im Wesentlichen ähnliches Volumen aufweisen;
- statistische Verarbeitung und Unterteilung von zwei oder mehr homogenen Klassen dreidimensionaler digitaler Blöcke in der digitalen Probe auf der Grundlage der Dichte der Schlüsselpunkte in jedem dreidimensionalen digitalen Block, wobei jede homogene Klasse (33, 33a und 33b) einen Teil der digitalen Probe (34) definiert, der dreidimensionale digitale Blöcke (28) mit ähnlicher Dichte an Schlüsselpunkten (6) und somit ähnlichen Gesteinseigenschaften aufweist;
- Auswählen mindestens eines repräsentativen Blocks (35) für jede homogene Klasse (33, 33a und 33b);
- Lokalisieren und Extrahieren (46) einer Teilprobe (2) aus der Gesteinsprobe (3), wobei die Teilprobe (2) mindestens einen der repräsentativen Blöcke (35) für jede homogene Klasse (33, 33a und 33b) umfasst.

2. Verfahren (1) nach Anspruch 1, **dadurch gekennzeichnet, dass** es umfasst:
- Durchführen einer weiteren Gesteinsanalyse, bei der jedes erste verarbeitete Bild (N_{key}) in eine Vielzahl von Interessenbereichen (7) unterteilt wird, die die Identifizierung der Gesteinsstruktur und/oder der Gesteinseigenschaften oder -merkmale im Bereich um jeden Schlüsselpunkt (6) ermöglichen;
- digitales Verarbeiten der ersten verarbeiteten Bilder (N_{key}), um die Schlüsselpunkte (6) in zwei oder mehr Gruppen (8, 8a und 8b) ähnlicher Punkte zu unterteilen, wobei die Gruppierung (20) für jedes erste verarbeitete Bild (N_{key}) ein erstes gruppiertes Bild (N_{Clust}) definiert und wobei die Gruppierung (20) auf der Grundlage einer Ähnlichkeit der digitalen Eigenschaften der ersten verarbeiteten Bilder (N_{key}) in den Interessenbereichen (7) erfolgt;
- Darstellen der Gesteinsprobe (3) durch eine zweite digitale Probe (18), bei der die Vielzahl der ersten gruppierten Bilder (N_{Clust}) entsprechend der Richtung T überlagert wird;
- Durchführen der volumetrischen Unterteilung (30) der zweiten digitalen Probe (18), wodurch die Vielzahl der dreidimensionalen digitalen Blöcke (28) definiert wird.

3. Verfahren nach Anspruch 1 oder 2, **gekennzeichnet durch**:
- Extrahieren des mindestens einen repräsentativen Blocks (35) aus jeder der zwei oder mehr homogenen Klassen (33, 33a und 33b) durch eine Auswahl (40) unter Verwendung statistischer Analysen und/oder Filterverfahren zum Sichten der digitalen 3D-Blöcke (28).

4. Verfahren nach Anspruch 3, **gekennzeichnet durch** die Bereitstellung einer Vorverarbeitung (11), die die Vielzahl der ersten 2D-Bilder (N_{slice}) digital verarbeitet und für jedes erste 2D-Bild (N_{slice}) ein entsprechendes erstes verarbeitetes 2D-Bild (Nₑₗ) ermittelt, wobei die Gesteinsanalyse (15) an der Vielzahl der ersten verarbeiteten 2D-Bilder (Nₑₗ) durchgeführt wird.

5. Verfahren nach Anspruch 1, **dadurch gekennzeichnet, dass** das Lokalisieren das Zuordnen (45) des mindestens einen repräsentativen Blocks (35) zu einer in homogene Klassen (33, 33a, 33b) unterteilten digitalen Probe (34, 34') umfasst und dass der mindestens eine repräsentative Block (35) in der Gesteinsprobe (3) mittels einer affinen Transformation identifiziert wird, um die Teilprobe (2) zu identifizieren.

6. Verfahren nach Anspruch 1, **dadurch gekennzeichnet, dass** das im Wesentlichen ähnliche Volumen durch eine statistische Analyse (25) der Dichte der in den dreidimensionalen digitalen Blöcken (28) enthaltenen Schlüsselpunkte (6) bestimmt wird oder dass das im Wesentlichen ähnliche Volumen einen minimalen Volumenwert (V1ₘᵢₙ) aufweist, der sich auf einen vordefinierten Wert bezieht.

7. Verfahren (100) zur Bestimmung der physikalischen oder petrophysikalischen Eigenschaften einer Gesteinsprobe (3), **gekennzeichnet durch**:
- Identifizieren und Extrahieren mindestens einer Teilprobe (2) aus der Gesteinsprobe (3) unter Verwendung eines oder mehrerer der Ansprüche 1 bis 6, wobei die mindestens eine Teilprobe (2) mindestens einen repräsentativen Block (35) für jede der zwei oder mehr homogenen Klassen (33, 33a und 33b) dreidimensionaler digitaler Blöcke (28) umfasst;
- digitales Erfassen (55) einer Vielzahl dritter digitaler Bilder (52) der mindestens einen Teilprobe (2) an dem mindestens einen repräsentativen Block (35) mit einer zweiten Auflösung, die höher ist als die erste Auflösung;
- Durchführen einer physikalischen oder petrophysikalischen Analyse (57) der Vielzahl der erfassten dritten digitalen Bilder (52), um die physikalischen oder petrophysikalischen Eigenschaften der Gesteinsprobe (3) ausgehend von den Eigenschaften des mindestens einen repräsentativen Blocks (35) für jede der zwei oder mehr homogenen Klassen (33) zu bestimmen.

8. Eine Teilprobe (2), die durch ein Verfahren nach einem oder mehreren der Ansprüche 1 bis 6 gewonnen wurde, **dadurch gekennzeichnet, dass** sie mindestens zwei repräsentative Blöcke (35) umfasst, wobei die mindestens zwei repräsentativen Blöcke (35) ein im Wesentlichen ähnliches Volumen aufweisen.

9. Ein Datenverarbeitungssystem, **dadurch gekennzeichnet, dass** es umfasst:
- einen Tomographen, der eingerichtet ist, einen ersten digitalen Scan (9) einer Gesteinsprobe (3) mit einer ersten Auflösung durchzuführen, wodurch eine Vielzahl von ersten 2D-Bildern (N_{slice}) definiert wird, und
- einen Prozessor, der eingerichtet ist, die Vielzahl erster 2D-Bilder (N_{slice}) zu erfassen und das Verfahren (1) zur Identifizierung einer für die Gesteinsprobe (3) repräsentativen Teilprobe (2) nach einem oder mehreren der Ansprüche 1 bis 6 auszuführen, und/oder dass es umfasst:
- einen weiteren Tomographen, der eingerichtet ist, einen zweiten digitalen Scan (51) der Teilprobe (2) mit einer zweiten Auflösung, die höher ist als die erste Auflösung, durchzuführen, wodurch eine Vielzahl von dritten 2D-Bildern (52) definiert wird, und
- einen weiteren Prozessor oder den genannten Prozessor, der eingerichtet ist, das Verfahren zur Bestimmung der physikalischen oder petrophysikalischen Eigenschaften einer Gesteinsprobe (3) nach Anspruch 7 auszuführen.

10. Computerprogramm, **dadurch gekennzeichnet, dass** es Anweisungen umfasst, die bewirken, dass der Computer bei Ausführung des Programms den Prozess nach einem oder mehreren der Ansprüche 1 bis 6 und/oder das Verfahren nach Anspruch 7 ausführt.

## Revendications

1. Procédé (1) d'identification d'un sous-échantillon (2) représentatif d'un échantillon de roche (3), qui comprend:
- l'acquisition numérique (10) à une première résolution d'une pluralité de premières images 2D (N_{slice}) dudit échantillon de roche (3);
- une analyse de roche (15) qui est effectuée par une analyse numérique des propriétés desdites premières images 2D pour déterminer une pluralité de points clés (6) sur chaque première image 2D et pour définir des premières images traitées correspondantes (N_{key}), les points clés (6) étant des descripteurs de texture;
- une superposition, selon la direction (T), desdites premières images traitées (N_{key}) pour l'obtention d'un échantillon de roche numérique traité (16);
- la subdivision par une subdivision volumétrique (30) de l'échantillon de roche numérique traité (16) en une pluralité de blocs numériques tridimensionnels (28) présentant un volume sensiblement similaire;
- le traitement statistique et la subdivision, sur la base de la densité des points clés dans chaque bloc numérique tridimensionnel, de deux ou plus de deux classes homogènes de blocs numériques tridimensionnels dans l'échantillon numérique, chaque classe homogène (33, 33a et 33b) définissant une partie d'échantillon numérique (34) comportant des blocs numériques tridimensionnels (28) présentant une densité similaire de points clés (6) et donc des propriétés de roche similaires;
- la sélection d'au moins un bloc représentatif (35) pour chaque classe homogène (33, 33a et 33b);
- la localisation et l'extraction (46) d'un sous-échantillon (2) dudit échantillon de roche (3), ledit sous-échantillon (2) comprenant au moins un desdits blocs représentatifs (35) pour chaque classe homogène (33, 33a et 33b).

2. Procédé (1) selon la revendication 1, **caractérisé en ce qu'**il comprend:
- l'exécution d'une analyse supplémentaire de roche par subdivision de chaque première image traitée (N_{key}) en une pluralité de régions d'intérêt (7) qui permettent l'identification de la structure de la roche et/ou des propriétés ou caractéristiques de la roche dans la zone entourant chaque point clé (6);
- le traitement numérique desdites premières images traitées (N_{key}) pour subdiviser les points clés (6) en deux ou plus de deux groupes (8, 8a et 8b) de points similaires, où le regroupement (20) définit une première image groupée (N_{Clust}) pour chaque première image traitée (N_{key}), et où le regroupement (20) est effectué sur la base d'une similarité des propriétés numériques des premières images traitées (N_{key}) dans les régions d'intérêt (7);
- la représentation dudit échantillon de roche (3) par un deuxième échantillon numérique (18) en superposant, selon la direction T, ladite pluralité de premières images groupées (N_{Clust});
- l'exécution de la subdivision volumétrique (30) dudit deuxième échantillon numérique (18) définissant la pluralité desdits blocs numériques tridimensionnels (28).

3. Procédé selon la revendication 1 ou 2, **caractérisé par**:
- l'extraction par une sélection (40) dudit au moins un bloc représentatif (35) à partir de chacune desdites deux ou plus de deux classes homogènes (33, 33a et 33b), à l'aide d'analyse statistique et/ou de méthodes de filtrage pour le criblage desdits blocs numériques 3D (28).

4. Procédé selon la revendication 3, **caractérisé en ce qu'**il prévoit un traitement préliminaire (11) qui traite numériquement ladite pluralité de premières images D (N_{slice}) et qui détermine pour chaque première image 2D (N_{slice}) une première image 2D traitée (Nₑₗ) correspondante, ladite analyse de roche (15) étant effectuée sur ladite pluralité de premières images 2D traitées (Nₑₗ).

5. Procédé selon la revendication 1, **caractérisé en ce que** la localisation comprend le mappage (45) dudit au moins un bloc représentatif (35) sur un échantillon numérique (34, 34') subdivisé en lesdites classes homogènes (33, 33a et 33b) et l'identification au moyen d'une transformation affine dudit au moins un bloc représentatif (35) dans ledit échantillon de roche (3) afin d'identifier ledit sous-échantillon (2).

6. Procédé selon la revendication 1, **caractérisé en ce que** ledit volume sensiblement similaire est déterminé par une analyse statistique (25) de la densité desdits points clés (6) inclus dans lesdits blocs numériques tridimensionnels (28) ou **en ce que** ledit volume sensiblement similaire présente une valeur minimale de volume (V1ₘᵢₙ) par référence à une valeur prédéfinie.

7. Une méthode (100) de détermination des propriétés physiques ou pétrophysiques d'un échantillon de roche (3), **caractérisé par**:
- l'identification et l'extraction à partir dudit échantillon de roche (3) d'au moins un sous-échantillon (2) à l'aide d'une ou de plusieurs des revendications 1 à 6, ledit au moins un sous-échantillon (2) comprenant au moins un bloc représentatif (35) pour chacune des deux ou plus de deux classes homogènes (33, 33a et 33b) de blocs numériques tridimensionnels (28);
- l'acquisition numérique (55), à une seconde résolution supérieure à ladite première résolution, d'une pluralité de troisièmes images numériques (52) dudit au moins un sous-échantillon (2) au niveau dudit au moins un bloc représentatif (35);
- l'exécution d'une analyse physique ou pétrophysique (57) de ladite pluralité de troisièmes images numériques (52) acquises pour déterminer les propriétés physiques ou pétrophysiques dudit échantillon de roche (3) à partir des propriétés dudit au moins un bloc représentatif (35) pour chacune desdites deux ou plus de deux classes homogènes (33).

8. Sous-échantillon (2) obtenu par un procédé selon une ou plusieurs des revendications 1 à 6, **caractérisé en ce qu'**il comprend au moins deux blocs représentatifs (35), lesdits au moins deux blocs représentatifs (35) ayant un volume sensiblement similaire.

9. Système de traitement de données, **caractérisé en ce qu'**il comprend:
- un tomographe configuré pour effectuer un premier balayage numérique (9) à une première résolution sur un échantillon de roche (3), définissant une pluralité de premières images 2D (N_{slice}), et
- un processeur configuré pour acquérir ladite pluralité de premières images 2D (N_{slice}), et pour exécuter le procédé (1) d'identification d'un sous-échantillon (2) représentatif dudit échantillon de roche (3) selon une ou plusieurs des revendications 1 à 6, et/ou comprend:
- un tomographe supplémentaire configuré pour exécuter un deuxième balayage numérique (51) à une seconde résolution supérieure à la première résolution du sous-échantillon (2), définissant une pluralité de troisièmes images 2D (52), et
- un processeur supplémentaire ou ledit processeur configuré pour exécuter le procédé de détermination des propriétés physiques ou pétrophysiques d'un échantillon de roche (3) selon la revendication 7.

10. Programme informatique, **caractérisé en ce qu'**il comprend des instructions qui, lorsque le programme est exécuté par un ordinateur, amènent l'ordinateur à exécuter le procédé selon une ou plusieurs des revendications 1 à 6 et/ou à exécuter le procédé selon la revendication 7.
